# FASCICULE DE BREVET EUROPEEN

(11) **EP 1 051 393 B1**
(45) Date de publication et mention de la délivrance du brevet: **01.10.2003**
(21) Numéro de dépôt: 98904231.2
(22) Date de dépôt: 27.01.1998
(51) Int. Cl.: C07C 309/15, C07C 309/24, C07F 9/30, A61K 31/185, A61K 31/66

(54) **NOUVEAUX DERIVES D'ACIDES AMINOALKANE SULFONIQUES, PHOSPHONIQUES ET PHOSPHINIQUES, LEUR PREPARATION ET LEUR UTILISATION COMME MEDICAMENTS**
DERIVATIVES OF AMINOALKANE SULPHONIC, PHOSPHONIC AND PHOSPHINIC ACIDS, THEIR PREPARATION AND THEIR USE AS MEDICAMENTS
NEW DERIVATIVES OF SULFONIC, PHOSPHONIC AND PHOSPHINIC AMINOALKANE ACIDS, THEIR PRODUCTION AND THEIR USE AS MEDICAMENTS

(43) Date de publication de la demande: 15.11.2000
(73) Titulaire: Merck Sante, 69008 Lyon (FR)
(72) Inventeur: BERTHELON, Jean-Jacques, F-69005 Lyon (FR); DURBIN, Philippe, F-69100 Villeurbanne (FR)
(74) Mandataire: Bernasconi, Jean Raymond
(86) Numéro de dépôt international: FR9800147
(87) Numéro de publication internationale: WO99037606

(56) Documents cités:
- FR-A- 2 457 281
- S. BUDAVARI, ET AL.: "THE MERCK INDEX, 12th Edition" 1996 , MERCK & CO. , WHITEHOUSE STATION, NJ, US XP002078934 voir page 4, monographie 14

## Description

La présente invention concerne des dérivés d'acides sulfoniques, phosphoniques et phosphiniques destinés au traitement de la dépendance à l'alcool et à d'autres substances.

Le brevet Japonais JP 7612093 décrit des composés de formule : 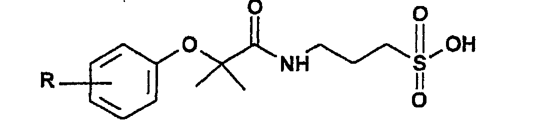 comme hypocholestérolémiants.

Le brevet Japonais JP 63201643 décrit l'utilisation de 4-palmitylsulfonate de potassium comme adjuvant dans les supports photographiques.

FR-A-2 457 281 a décrit des sels de l'acétyl homotaurine comme stabilisateurs membranaires. Le sel de calcium de l'acéthylhomotaurine est utilisé dans le traitement de l'alcoolisme (sous le nom d'acamprosate).

La présente invention a pour objet de nouveaux dérivés d'acides sulfoniques, phosphoniques et phosphiniques représentés par la formule (I): 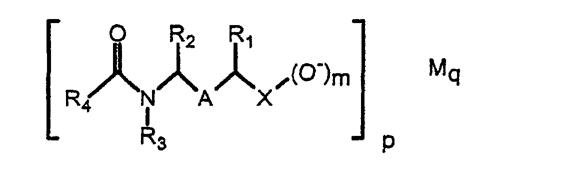 dans laquelle
X représente 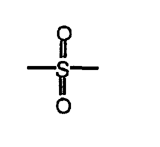 R₁, R₂, R₃ sont choisis parmi l'hydrogène et un radical alkyle en C₁-C₇
A représente un groupement de formule 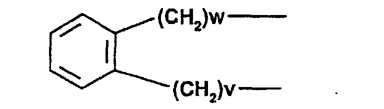 avec v et w = 0, 1, 2
ou un groupement de formule

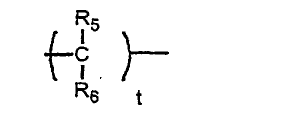

R₅, R₆ étant choisis indépendamment l'un de l'autre parmi l'hydrogène, un radical alkyle en C₁-C₇, un radical aryle ayant de 6 à 14 atomes de carbone et un radical hétéroaryle choisi parmi furyle, thiényle et thiazolyle, les radicaux aryle et hétéroaryle pouvant porter 1 à 3 substituants choisis parmi un groupe alkyle en C₁-C₇, un halogène ou un groupe trifluorométhyle, et t = 1-3.
R₄ est choisi parmi l'hydrogène, un radical alkyle en C₁-C₇, un radical CF₃, un radical aryle ayant de 6 à 14 atomes de carbone et un radical hétéroaryle choisi parmi furyle, thiényle et thiazolyle, les radicaux aryle et hétéroaryle pouvant porter 1 à 3 substituants choisis parmi un groupe alkyle en C₁-C₇, un halogène ou un groupe trifluorométhyle,
M représente un métal monovalent (Na, K, Li) ou un métal divalent (Ca, Mg, Sr, Zn), m = 1 ou 2,
p = 1 - 2 et q = 1 - 2, p et q étant tels que la neutralité électrique du sel soit assurée,
R₄ n'étant pas un radical méthyle lorsque R₁, R₂ et R₃ représentent l'hydrogène.

Les composés de l'invention peuvent contenir des centres chiraux. Les isomères optiques, les racémiques, les énantiomères, les diastéroisomères font partie de l'invention.

La demanderesse a montré que cette famille de produits permettent de diminuer la consommation d'alcool chez le rat présentant une dépendance alcoolique. Leurs applications thérapeutiques concement entre autre le domaine de la dépendance à l'alcool et à d'autres substances susceptibles d'entrainer une accoutumance comme par exemple les dérivés opiacés, les nicotiniques, les caféiniques, les amphétamines, les cannabinoïdes, les tranquillisants.

La présente invention s'étend aussi aux compositions pharmaceutiques renfermant comme principe actif un des composés de formule (I) éventuellement en combinaison avec un ou plusieurs excipients ou véhicules pharmaceutiquement acceptables.

Parmi les compositions selon l'invention on pourra citer, à titre d'exemple et de façon non limitative les comprimés, gélules, solutions buvables, capsules.

Les composés de l'invention peuvent être administrés à doses comprises entre 0,01 g et 1 g de une à trois fois par jour.

Parmi les composés préférés de la formule 1 on peut citer par exemple :
3-(2-(méthyl)propanoylamino)-propanesulfonate de calcium
3-(2-(méthyl)propanoylamino)-propanesulfonate de magnésium
3-(butanoylamino)-propanesulfonate de calcium
3-(butanoylamino)-propanesulfonate de magnésium
3-(pentanoylamino)-propanesulfonate de calcium
3-(benzoylamino)-propanesulfonate de calcium
3-(benzoylamino)-propanesulfonate de magnésium
3-(2-(méthyl)propanoylamino)-propanesulfonate de zinc
3-(2-(méthyl)propanoylamino)-propanesulfonate de strontium
3-(3-(méthyl)butanoylamino)-propanesulfonate de calcium
3-(3-(méthyl)butanoylamino)-propanesulfonate de magnésium
3-(2-2-(diméthyl)propanoylamino)-propanesulfonate de calcium
3-(2-2-(diméthyl)propanoylamino)-propanesulfonate de magnésium
3-(acétylamino)-2-méthyl-propanesulfonate de calcium
3-(acétylamino)-3-méthyl-propanesulfonate de calcium
3-(acétylamino)-3-méthyl-propanesulfonate de magnésium
3-(acétylamino)-1-méthyl-propanesulfonate de calcium
3-(acétylamino)-2-phényl-propanesulfonate de calcium
2-(2-acétylaminométhyl)-phénylméthanesulfonate de calcium
N-méthyl-3-(acétylamino)-propanesulfonate de calcium
3-(acétylamino)-2-2-diméthyl-propanesulfonate de calcium
3-(trifluorométhylcarbonyl)-propanesulfonate de calcium.

On préfère tout particulièrement les composés de formule I dans lesquels R₄ est un radical alkyle en C₂-C₇ et notamment un radical ramifié.

Les composés suivants font également partie de l'invention :
Acide 3-((2-méthyl)-propanoylamino)-propanesulfonique
Acide 3-(butanoylamino)-propanesulfonique
Acide 3-(pentanoylamino)-propanesulfonique
Acide 3-(benzoylamino)-propanesulfonique
Acide N-méthyl-3-(acétylamino)propanesulfonique
Acide 3-((3-méthyl)butanoylamino)-propanesulfonique
Acide 3-((2-2-diméthyl)propanoylamino)-propanesulfonique
Acide 3-(acétylamino)-2-méthyl-propanesulfonique
Acide 3-(acétylamino)-3-méthyl-propanesulfonique
Acide 3-(acétylamino)-1-méthyl-propanesulfonique
Acide 3-(acétylamino)-2-phényl-propanesulfonique
Acide 2-(2-acétylaminométhyl)-phénylméthanesulfonique
Acide 3-(acétylamino)-2-2-diméthyl-propanesulfonique
Acide 3-(trifluorométhylcarbonoyl)-propanesulfonique

L'invention vise également un procédé de préparation des composés de l'invention. Celui-ci est résumé dans le schéma 1.

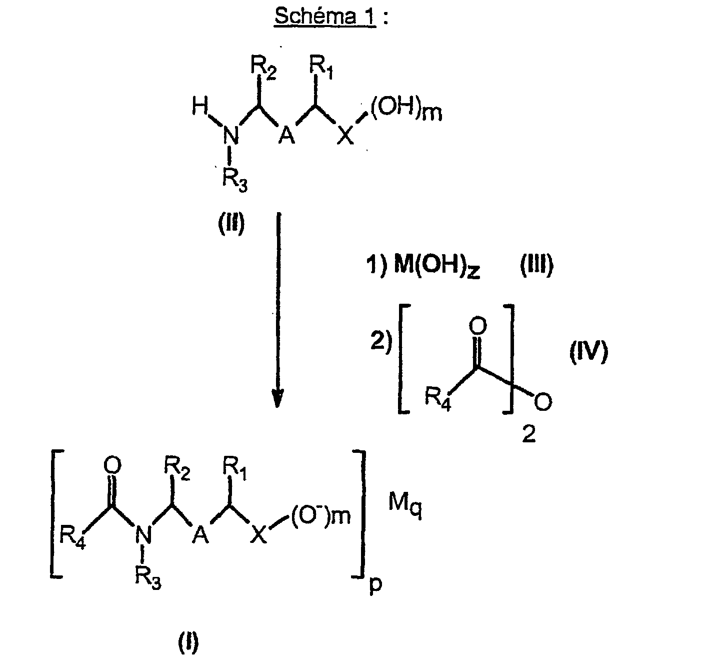

La réaction peut être effectuée en faisant réagir le composé de formule (II) avec la base M(OH)_{z} où z est la valence de M puis, en maintenant à une température comprise entre 15° C et 20° C, on ajoute l'anhydride de formule (IV). On laisse réagir une nuit et après traitement on obtient le composé de formule (1).

La liste des exemples suivants illustrant l'invention n'est pas limitative. Dans les données de résonnance magnétique nucléaire du proton (RMN¹H), les abréviations suivantes ont été employées :
- ppm pour parties par million
- s pour singulet
- d pour doublet
- t pour triplet
- q pour quadruplet
- m pour massif complex
- j pour les couplages exprimés en Hertz
- dd pour doublet de doublet

### Exemple 1

### 3-(2-2-(diméthyl)propanoylamino)-propanesulfonate de calcium

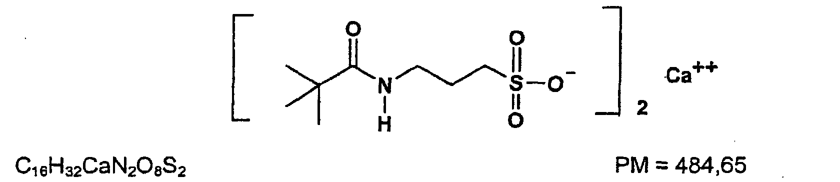

A une solution de 22,3 g (0,1 mole) d'acide aminopropanesulfonique dans une quantité suffisante d'eau distillée, on ajoute 8,1 g (0,11 mole) de Ca(OH)₂. On obtient une suspension blanche que l'on maintient sous agitation 15 minutes.

On refroidit à 15° C et on ajoute goutte à goutte 35,2 g (0,2 mole) d'anhydride (2-2-diméthyl)propanoïque en maintenant la température entre 15° C et 20° C. On porte ensuite sous agitation une nuit à température ambiante. La solution obtenue est ensuite évaporée sous vide et le résidu est repris avec qs d'eau distillée pour le solubiliser. On ajoute à nouveau 17,6 g (0,1 mole) d'anhydride (2-2-diméthyl)propanoïque entre 15° C et 20° C puis on laisse à nouveau une nuit sous agitation à température ambiante. On évapore à sec sous vide. Le résidu est repris par 300 ml d'éthanol absolu contenant 1,5 ml d'acide chlorhydrique concentré. Le précipité obtenu est essoré, séché. On le reprend ensuite par la quantité d'eau distillée nécessaire à sa dissolution. Après lavage à l'éther, on additionne lentement à la phase aqueuse de l'acétone jusqu'à l'obtention d'un trouble persistant. On poursuit l'agitation jusqu'à fin de précipitation, on essore et on sèche.
Poids obtenu : 4,5 g (Rdt : 37 %)
PF_{G} : 300° C
IR_{γC=O} : 1623 cm⁻¹
¹HRMN (D₂O) δ en ppm : 0,83 (s, 3CH₃) ; 1,59 (m, CH₂) ; 2,56 (m, CH₂); 2,97 (m, CH₂).

| Analyse pondérale : (C₁₆H₃₂CaN₂O₈S₃ ; 0.25H₂O) | | | | | |
|---|---|---|---|---|---|
| | C% | H% | Ca% | N% | S% |
| Calculée | 39,65 | 6,66 | 8,27 | 5,78 | 13,23 |
| Trouvée | 38,72 | 6,61 | 8,49 | 5,87 | 13,33 |

### Exemple 2

### 3-(2-(méthyl)propanoylamino)-propanesulfonate de calcium

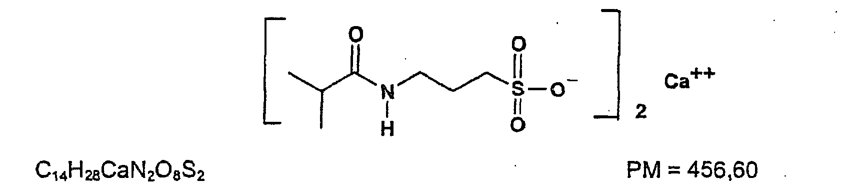

PF_{G} > 360° C
IR_{γC=O} : 1644 cm⁻¹
¹HRMN (D₂O) δ en ppm : 1,1 (d, 2CH₃) ; 1,93 (m, CH₂) ; 2,48 (m, CH₂) ; 2,90 (m, CH₂); 3,29 (t, CH₂)

| Analyse pondérale: | | | | | |
|---|---|---|---|---|---|
| | C % | H % | Ca% | N% | S% |
| Calculée | 36,83 | 6,18 | 8,78 | 6,14 | 14,04 |
| Trouvée | 36,96 | 6,27 | 8,70 | 6,27 | 14,25 |

### Exemple 3

### 3-(2-(méthyl)propanoylamino)-propanesulfonate de magnésium

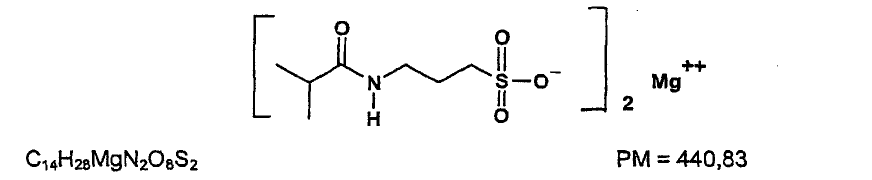

PF_{G}: 270 - 273° C
IR_{γC=O}: 1644 cm⁻¹
¹HRMN (D₂O) δ en ppm : 0,95 (d, 2CH₃) ; 1,78 (m, CH₂) ; 2,34 (m, CH₂) ; 2,76 (m, CH₂); 3,14 (t, CH₂)

| Analyse pondérale : | | | | | |
|---|---|---|---|---|---|
| | C% | H% | Mg% | N% | S% |
| Calculée | 36,65 | 6,59 | 5,30 | 6,11 | 13,97 |
| Trouvée | 36,56 | 6,60 | 5,52 | 6,15 | 13,57 |

### Exemple 4

### 3-(butanoylamino)-propanesulfonate de calcium

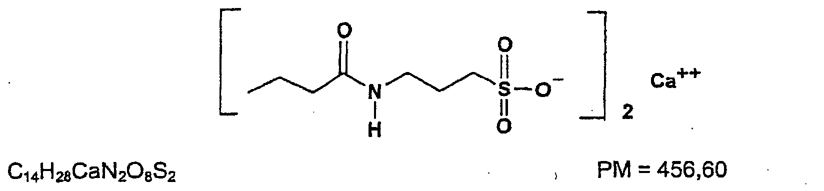

PF_{G} > 360° C
IR_{γC=O}: 1633 cm⁻¹
¹HRMN (D₂O) δ en ppm : 0,81 (t, CH₃) ; 1,49 (m, CH₂) ; 1,84 (m, CH₂) ; 2,12 (t, CH₂); 2,83 (m, CH₂) ; 3,21 (t, CH₂)

| Analyse pondérale: | | | | | |
|---|---|---|---|---|---|
| | C% | H% | Ca% | N% | S% |
| Calculée | 36,83 | 6,18 | 8,78 | 6,14 | 14,04 |
| Trouvée | 36,84 | 6,23 | 8,79 | 6,30 | 14,29 |

### Exemple 5

### 3-(butanoylamino)-propanesulfonate de magnésium

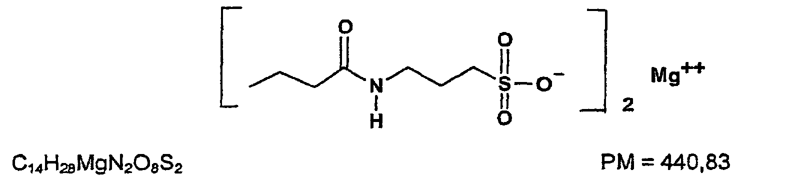

PF_{G} : 325° C
IR_{γC=O}: 1635 cm⁻¹
¹HRMN (D₂O) δ en ppm : 0,94 (t, CH₃) ; 1,64 (m, CH₂) ; 1,98 (m, CH₂) ; 2,26 (t, CH₂); 2,97 (m, CH₂) ; 3,35 (t, CH₂)

| Analyse pondérale : (C₁₄H₂₈MgN₂O₈S₂; 2H₂O) | | | | | |
|---|---|---|---|---|---|
| | C% | H% | Mg% | N% | S% |
| Calculée | 35,26 | 6,76 | 5,10 | 5,38 | 13,45 |
| Trouvée | 35,11 | 6,62 | 5,35 | 5,90 | 13,10 |

### Exemple 7

### 3-(pentanoylamino)-propanesulfonate de calcium

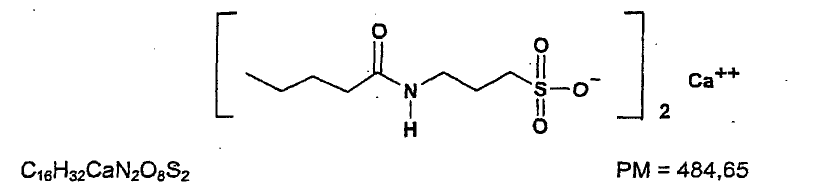

PF_{G} > 360° C
IR_{γC=O} : 1633 cm⁻¹
¹HRMN (D₂O) δ en ppm : 0,99 (t, CH₃) ; 1,4 (m, CH₂) ; 1,67 (m, CH₂) ; 2,04 (m, CH₂); 2,35 (t, CH₂) ; 3,03 (m, CH₂) ; 3,41 (t, CH₂)

| Analyse pondérale : | | | | | |
|---|---|---|---|---|---|
| | C% | H% | Ca% | N% | S% |
| Calculée | 39,65 | 6,66 | 8,27 | 5,78 | 13,23 |
| Trouvée | 39,75 | 6,75 | 8,33 | 5,54 | 13,23 |

### Exemple 8

### 3-(benzoylamino)-propanesulfonate de calcium

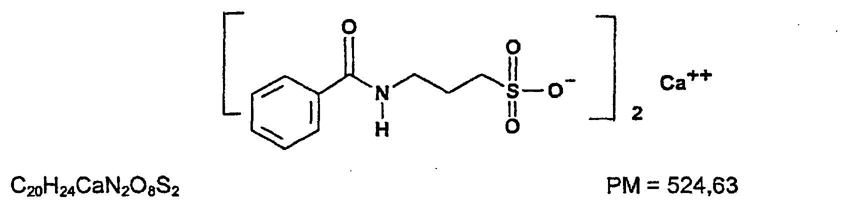

PF_{G} > 360° C
IR_{γC=O} : 1637 cm⁻¹
¹HRMN (D₂O) δ en ppm : 1,78 (m, CH₂) ; 2,72 (m, CH₂); 3,21 (t, CH₂) ; 7,2-7,45 (m, 5AR)

| Analyse pondérale : (C₂₀H₂₄CaN₂O₈S₂ ; 1H₂O) | | | | | |
|---|---|---|---|---|---|
| | C% | H% | Ca% | N% | S% |
| Calculée | 44,27 | 4,83 | 7,39 | 5,16 | 11,82 |
| Trouvée | 43,98 | 4,75 | 7,23 | 5,11 | 11,42 |

### Exemple 9

### 3-(benzoylamino)-propanesulfonate de magnésium

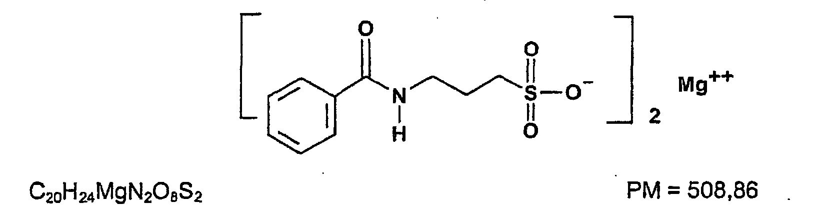

PF_{G} : 350° C
IR_{γC=O} : 1640 cm⁻¹
¹HRMN (D₂O) δ en ppm : 1,9 (m, CH₂) ; 2,83 (m, CH₂) ; 3,33 (t, CH₂) ; 7,32-7,68 (m, 5AR)

| Analyse pondérale : (C₂₀H₂₄MgN₂O₈S₂ ; 2H₂O) | | | | | |
|---|---|---|---|---|---|
| | C% | H% | Mg% | N% | S% |
| Calculée | 44,08 | 5,18 | 4,46 | 5,14 | 11,77 |
| Trouvée | 44,49 | 5,18 | 4,48 | 5,16 | 11,42 |

### Exemple 11

### 3-(2-(méthyl)propanylamino)-propanesulfonate de zinc

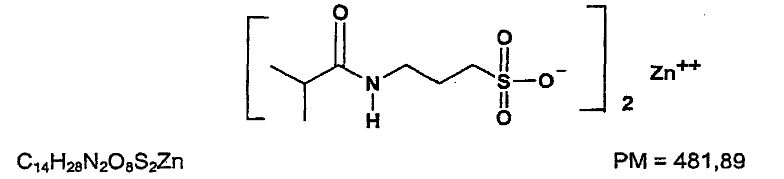

PF_{G}: 114°C
IR_{γC=O} : 1637 cm⁻¹
¹HRMN (D₂O) δ en ppm : 0,77 (d, CH₃) ; 1,6 (m, CH₂); 2,17 (m, CH) ; 2,58 (m, CH₂); 2,97 (t, CH₂)

| Analyse pondérale : (C₁₄H₂₈N₂O₈S₂Zn; 2H₂O) | | | | | |
|---|---|---|---|---|---|
| | C% | H% | N% | S% | Zn% |
| Calculée | 32,46 | 6,27 | 5,41 | 12,38 | 12,62 |
| Trouvée | 32,46 | 6,27 | 5,30 | 12,38 | 12,44 |

### Exemple 12

### 3-(2-(méthyl)propanoylamino)-propanesulfonate de strontium

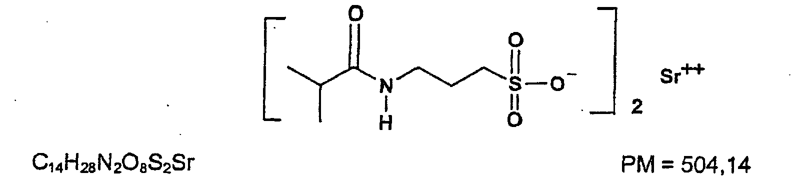

PF_{G} : 345-350° C
IR_{γC=O} : 1642 cm⁻¹
¹HRMN (D₂O) δ en ppm : 1 (d, CH₃) ; 1,83 (m, CH₂) ; 2,39 (m, CH) ; 2,8 (m, CH₂); 3,19 (t, CH₂)

| Analyse pondérale : | | | | | |
|---|---|---|---|---|---|
| | C% | H% | N% | S% | Sr% |
| Calculée | 33,36 | 5,60 | 5,56 | 12,72 | 17,38 |
| Trouvée | 33,12 | 5,62 | 5,24 | 12,24 | 17,85 |

### Exemple 13

### 3-(3-(méthyl)butanoylamino)-propanesulfonate de calcium

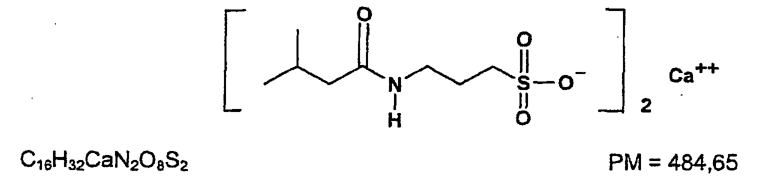

PF_{G} > 350° C
IR_{γC=O} : 1633 cm⁻¹
'HRMN (D₂O) δ en ppm : 0,91 (d, 2CH₃) ; 1,89-2,12 (m, 2CH₂ + CH) ; 2,92 (m, CH₂); 3,3 (t, CH₂)

| Analyse pondérale : | | | | | |
|---|---|---|---|---|---|
| | C% | H% | Ca% | N% | S% |
| Calculée | 39,65 | 6,66 | 8,27 | 5,78 | 13,23 |
| Trouvée | 39,07 | 6,41 | 8,37 | 5,83 | 13,08 |

### Exemple 14

### 3-(3-(méthyl)butanoylamino)-propanesulfonate de magnésium

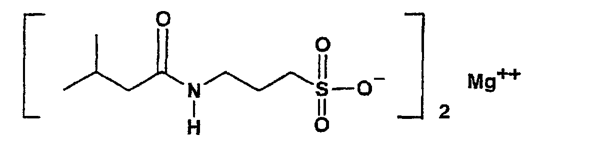

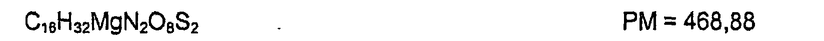

PF_{G} : 280-287° C
IR_{γC=O}: 1644 cm⁻¹
¹HRMN (D₂O) δ en ppm : 0,66 (d, 2CH₃) ; 1,63-1,87 (m, 2CH₂ + CH) ; 2,67 (m, CH₂) ; 3,05 (t, CH₂)

| Analyse pondérale : (C₁₆H₃₂MgN₂O₈S₂, 2H₂O) | | | | | |
|---|---|---|---|---|---|
| | C% | H% | Mg% | N% | S% |
| Calculée | 38,05 | 7,18 | 4,81 | 5,55 | 12,70 |
| Trouvée | 38,40 | 7,10 | 5,53 | 5,67 | 13,13 |

### Exemple 15

### 3-(2,2-(diméthyl)propanoylamino)-propanesulfonate de magnésium

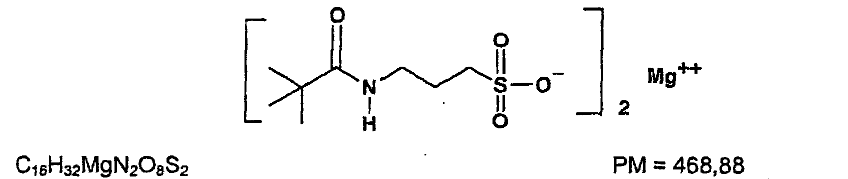

PF_{G} : 200-250° C
IR_{γC=O} : 1630 cm⁻¹
¹HRMN (D₂O) δ en ppm : 1,28 (s, 3CH₃) ; 2,04 (m, CH₂) ; 3,02 (m, CH₂) ; 3,42 (t, CH₂)

| Analyse pondérale : (C₁₆H₃₂MgN₂O₈S₂, 5H₂O) | | | | | |
|---|---|---|---|---|---|
| | C% | H% | Mg% | N% | S% |
| Calculée | 34,42 | 7,57 | 4,35 | 5,04 | 11,49 |
| Trouvée | 33,94 | 7,48 | 4,35 | 5,38 | 11,68 |

### Exemple 16

### 3-(acétylamino)-2-méthyl-propanesulfonate de calcium

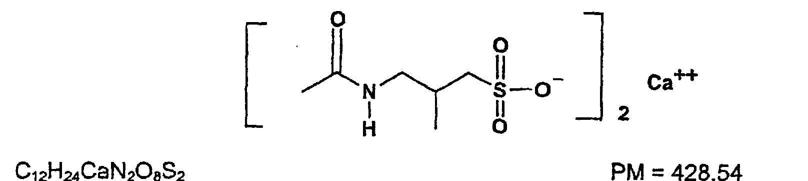

PF_{G}: 270° C
IR_{γC=O}: 1638 cm⁻¹
¹HRMN (D₂O) δ en ppm : 1,15 (d, CH₃) ; 2,07 (s, CH₃) ; 2,25 (m, CH) ; 2,83 (m, CH) ; 3,02 (m, CH) ; 3,24 (n, CH₂)

| Analyse pondérale : (C₁₂H₂₄CaN₂O₈S₂, 0,5H₂O) | | | | | |
|---|---|---|---|---|---|
| | C% | H% | Ca% | N% | S% |
| Calculée | 33,63 | 5,65 | 9,35 | 6,54 | 14,96 |
| Trouvée | 32,41 | 5,74 | 9,28 | 6,27 | 14,47 |

### Exemple 17

### 3-(acétylamino)-3-méthyl-propanesultonate de calcium

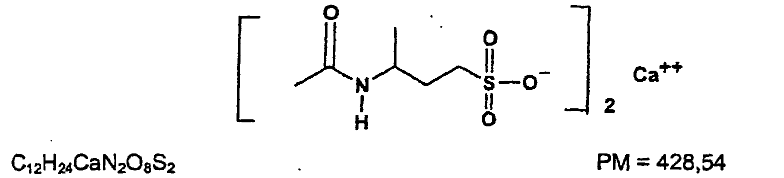

PF_{G} : 275-285° C
IR_{γC=O} : 1364 cm⁻¹
¹HRMN (D₂O) δ en ppm : 1,15 (d, CH₃) ; 1,85 (m, CH₂) ; 1,98 (s, CH₃) ; 2,91 (t, CH₂) ; 3,94 (m, CH)

| Analyse pondérale : (C₁₂H₂₄CaN₂O₈S₂, 0,5H₂O) | | | | | |
|---|---|---|---|---|---|
| | C% | H% | Ca% | N% | S% |
| Calculée | 32,96 | 5,76 | 9,17 | 6,41 | 14,66 |
| Trouvée | 32,61 | 5,79 | 8,95 | 6,34 | 14,29 |

### Exemple 18

### 3-(acétylamino)-3-méthyl-propaneslfonate de magnésium

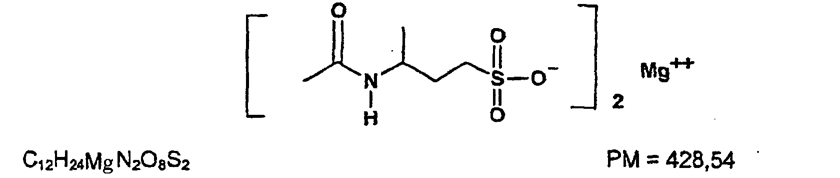

¹HRMN (D₂O) δ en ppm : 1,1 (d, CH₃); 1,78 (m, CH₂) ; 1,9 (s, CH₃) ; 2,84 (t, CH₂) ; 3,85 (m, CH)

### Exemple 19

### 3-(acétylamino)-1-méthyl-propanesulfonate de calcium

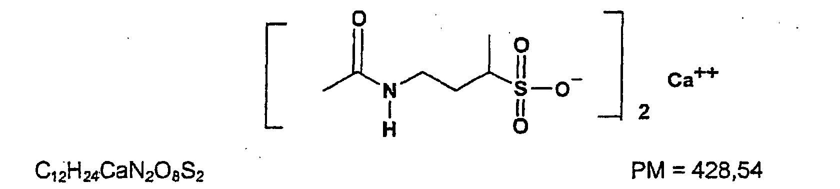

PF_{G} > 360° C
IR_{γC=O} : 1670 cm⁻¹
¹HRMN (D₂O) δ en ppm : 1,44 (d, CH₃) ; 1,77 (m, CH) ; 2,11 (s, CH₃) ; 2,33 (m, CH) ; 3,03 (m, CH) ; 3,45 (m, CH₂)

| Analyse pondérale : | | | | | |
|---|---|---|---|---|---|
| | C% | H% | Ca% | N% | S% |
| Calculée | 33,63 | 5,65 | 9,35 | 6,54 | 14,96 |
| Trouvée | 33,34 | 5,67 | 9,35 | 6,50 | 15,06 |

### Exemple 20

### 2-(2-acétylaminométhyl)-phénylméthanesulfonate de calcium

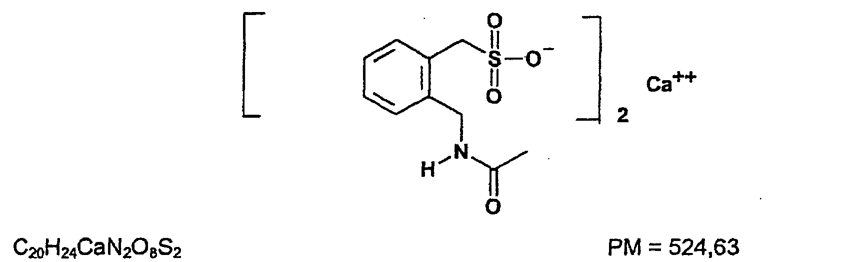

PF_{G}: 260-265° C
IR_{γC=O}: 1640 cm⁻¹
¹HRMN (D₂O) δ en ppm : 2 (s, CH₃); 4,26 (m, CH₂); 4,48 (s, CH₂); 7,3-7,4 (m, 4AR)

| Analyse pondérale : (C₂₀H₂₄CaN₂O₈S₂; 1H₂O) | | | | | |
|---|---|---|---|---|---|
| | C% | H% | Ca% | N% | S% |
| Calculée | 44,26 | 4,83 | 7,38 | 5,16 | 11,81 |
| Trouvée | 44,45 | 4,80 | 7,63 | 5,23 | 11,25 |

### Exemple 21

### N-méthyl-3-(acétylamino)-propanesulfonate de calcium

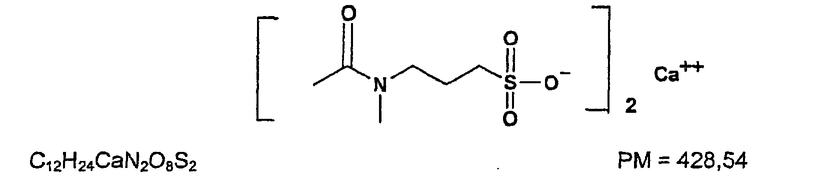

IR_{γC=O} : 1611 cm⁻¹
¹HRMN (D₂O) δ en ppm : 2 (m, CH₂) ; 2,1 (s, CH₃); 2,9 (m, CH₂); 3,06 (s, CH₃); 3,48 (n, CH₂)

### Exemple 23

### 3-(acétylamino)-2-phényl-propanesulfonate de calcium

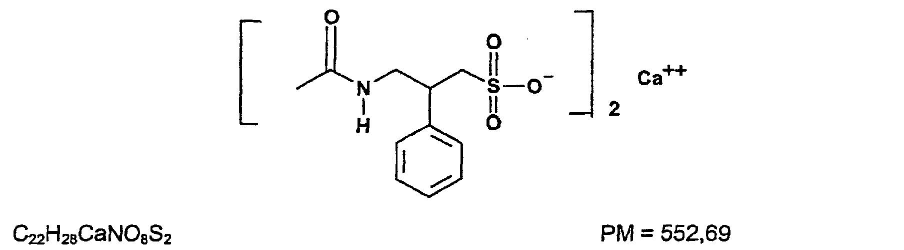

PF_{G}: 240-250° C
IR_{γC=O}: 1636 cm⁻¹
¹HRMN (D₂O) δ en ppm : 1,88 (s, CH₃) ; 3,28-3,48 (m, 2CH₂); 3,59-3,66 (m, CH) ; 7,33-7,46 (m, 5AR)

| Analyse pondérale : (C₂₂H₂₈CaN₂O₈S₂, 1H₂O) | | | | | |
|---|---|---|---|---|---|
| | C% | H% | Ca% | N% | S% |
| Calculée | 46,33 | 5,30 | 7,02 | 4,91 | 11,24 |
| Trouvée | 46,66 | 5,04 | 7,23 | 4,96 | 10,36 |

On donnera ci-après les résultats d'une étude pharmacologique des composés de l'invention.

### Consommation d'alcool chez les rats dépendants:

Des rats de souche Long-Evans et pesant 200 g à la mise en place de l'essai sont isolés en cage individuelle. Pour établir la dépendance alcoolique, on leur donne pour seule boisson une solution d'eau alcoolisée à 10 % (V/V) pendant 3 semaines. La nourriture leur est fournie ad-libitum.

A l'issue de cette période de 3 semaines, on offre à l'animal pendant 2 semaines le choix entre de l'eau et de l'eau alcoolisée. Seuls les rats consommant plus de 3 g/kg d'alcool par jour sont conservés pour la suite de l'essai.

A l'issue de cette période, on administre le produit à étudier par voie intrapéritonéale à la dose de 100 mg/kg/j pendant deux semaines sur des lots de 5 à 8 rats. Un lot témoin reçoit de l'eau physiologique par voie intrapéritonéale. Tous les rats ont libre choix entre l'eau et la solution alcoolisée, et la nourriture est ad-libitum.

Les consommations d'eau et de solution alcoolisée sont notées avant et pendant le traitement et rapportées au poids de l'animal.

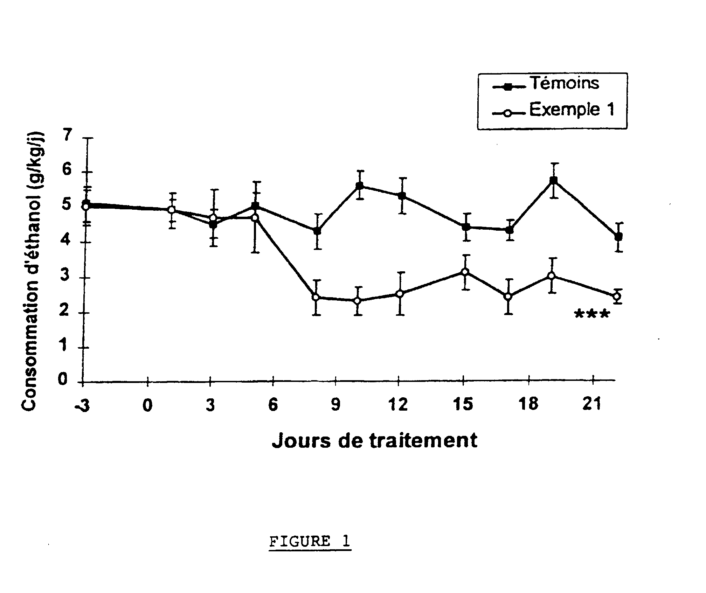

A titre d'exemple, on a représenté sur la figure 1 l'effet du composé de l'exemple 1 sur la consommation d'alcool.

Dans des essais in vitro, il a par ailleurs été montré que ces composés avaient la capacité de déplacer l'acétylhomotaurinate de calcium tritié à partir d'une préparation de coupe de cerveau de rat.

| **Exemple** | **IC50 (µM)** |
|---|---|
| 1 | 46,9 |
| 3 | 28,9 |
| 14 | 42 |
| 15 | 49,5 |
| 17 | 93 |

## Revendications

1. Composés de formule 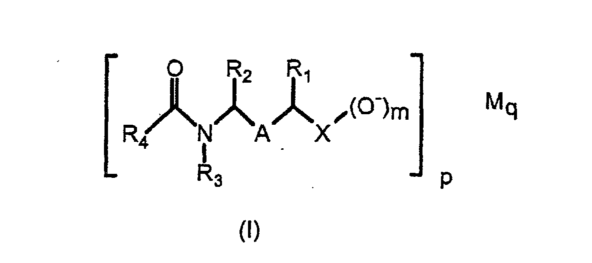 dans laquelle
X représente 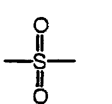 R₁, R₂, R₃ sont choisis parmi l'hydrogène et un radical alkyle en C₁-C₇
A représente un groupement de formule 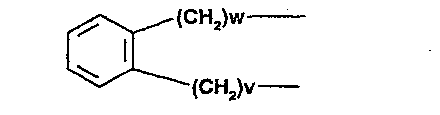 avec v et w = 0, 1, 2
ou un groupement de formule 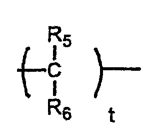 R₅, R₆ étant choisis indépendamment l'un de l'autre parmi l'hydrogène, un radical alkyle en C₁-C₇, un radical aryle ayant de 6 à 14 atomes de carbone et un radical hétéroaryle choisi parmi furyle, thiényle et thiazolyle, les radicaux aryle et hétéroaryle pouvant porter 1 à 3 substituants choisis parmi un groupe alkyle en C₁-C₇, un halogène ou un groupe trifluorométhyle, et t = 1-3.
R₄ est choisi parmi l'hydrogène, un radical alkyle en C₁-C₇, un radical CF₃, un radical aryle ayant de 6 à 14 atomes de carbone et un radical hétéroaryle choisi parmi furyle, thiényle et thiazolyle, les radicaux aryle et hétéroaryle pouvant porter 1 à 3 substituants choisis parmi un groupe alkyle en C₁-C₇, un halogène ou un groupe trifluorométhyle,
M représente un métal monovalent (Na, K, Li) ou un métal divalent (Ca, Mg, Sr, Zn),
m = 1 ou 2,
p = 1 - 2 et q = 1 - 2, p et q étant tels que la neutralité électrique du sel soit assurée,
R₄ n'étant pas un radical méthyle lorsque R₁, R₂ et R₃ représentent l'hydrogène.

2. Les composés selon la revendication 1, choisis parmi les composés suivants :
3-(2-(méthyl)propanoylamino-propanesulfonate de calcium
3-(2-(méthyl)propanoylamino)-propanesulfonate de magnésium
3-(butanoylamino)-propanesulfonate de calcium
3-(butanoylamino)-propanesulfonate de magnésium
3-(pentanoylamino)-propanesulfonate de calcium
3-(benzoylamino)-propanesulfonate de calcium
3-(benzoylamino)-propanesulfonate de magnésium
3-(2-(méthyl)propanoylamino)-propanesulfonate de zinc
3-(2-(méthyl)propanoylamino)-propanesulfonate de strontium
3-(3-(méthyl)butanoylamino)-propanesulfonate de calcium
3-(3-(méthyl)butanoylamino)-propanesulfonate de magnésium
3-(2-2-(diméthyl)propanoylamino)-propanesulfonate de calcium
3-(2-2-(diméthyl)propanoylamino)-propanesulfonate de magnésium
3-(acétylamino)-2-méthyl-propanesulfonate de calcium
3-(acétylamino)-3-méthy!-propanesulfonate de calcium
3-(acétylamino)-3-méthyl-propanesulfonate de magnésium
3-(acétylamino)-1-méthyl-propanesulfonate de calcium
3-(acétylamino)-2-phényl-propanesulfonate de calcium
2-(2-acétylaminométhyl)-phénylméthanesulfonate de calcium
N-méthyl-3-(acétylamino)-propanesulfonate de calcium
3-(acétylamino)-2-2-diméthyl-propanesulfonate de calcium
3-(trifluorométhylcarbonoyl)-propanesulfonate de calcium

3. Procédé de préparation des composés de formule I selon la revendication 1, qui consiste à faire réagir un composé de formule II : 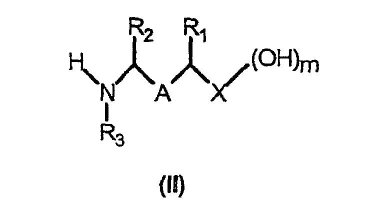 avec un composé de formule III :
M(OH)_{z} (III)
z étant la valence du métal M,
puis avec un composé de formule IV :

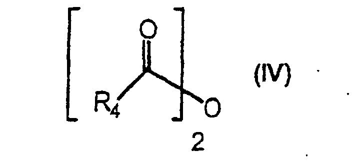

4. Composition pharmaceutique contenant un composé selon l'une quelconque des revendications 1 à 3.

## Patentansprüche

1. Verbindungen mit der Formel 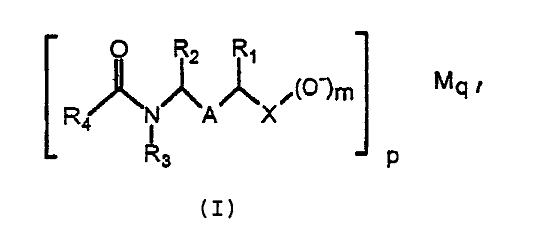 in welcher
- X 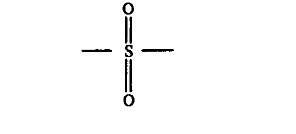 bedeutet,
- R₁, R₂ und R₃ aus Wasserstoff und einem C₁- bis C₇-Alkylrest ausgewählt sind und
- A eine Gruppierung mit der Formel 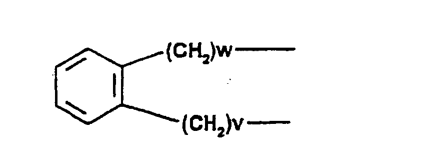 mit v und w = 0, 1 und 2, oder
eine Gruppierung mit der Formel 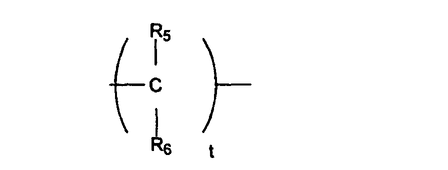 bedeutet, worin
- R₅ und R₆ unabhängig voneinander aus Wasserstoff, einem C₁- bis C₇-Alkylrest, einem Arylrest mit 6 bis 14 Kohlenstoffatomen und einem aus Furyl, Thienyl und Thiazolyl ausgewählten Heteroarylrest ausgewählt sind, wobei der Aryl- und der Heteroarylrest 1 bis 3 Substituenten tragen kann, die aus einer C₁- bis C₇-Alkylgruppe, einem Halogenatom oder einer Trifluormethylgruppe ausgewählt sind, und t = 1 bis 3,
- R₄ aus Wasserstoff, einem C₁- bis C₇-Alkylrest CF₃, einem Arylrest mit 6 bis 14 Kohlenstoffatomen und einem aus Furyl, Thienyl und Thiazolyl ausgewählten Heteroarylrest ausgewählt ist, wobei der Aryl- und der Heteroarylrest 1 bis 3 Substituenten tragen kann, die aus einer C₁- bis C₇-Alkylgruppe, einem Halogenatom oder einer Trifluormethylgruppe ausgewählt sind, und
- M ein einwertiges Metall (Na, K, Li) oder ein zweiwertiges Metall (Ca, Mg, Sr, Zn) bedeutet,
- m = 1 oder 2,
- p = 1 bis 2 und q = 1 bis 2, wobei p und q derart sind, dass die elektrische Neutralität des Salzes sichergestellt bleibt, und
- R₄ keinen Methylrest bedeutet, wenn R₁, R₂ und R₃ Wasserstoff bedeuten.

2. Verbindungen nach Anspruch 1, die aus folgenden Verbindungen ausgewählt sind:
Calcium-3-(2-(methyl)propanoylamino)-propansulfonat
Magnesium-3-(2-(methyl)propanoylamino)-propansulfonat
Calcium-3-(butanoylamino)-propansulfonat
Magnesium-3-(butanoylamino)-propansulfonat
Calcium-3-(pentanoylamino)-propansulfonat
Calcium-3-(benzoylamino)-propansulfonat
Magnesium-3-(benzoylamino)-propansulfonat
Zink-3-(2-(methyl)propanoylamino)-propansulfonat
Strontium-3-(2-(methyl)propanoylamino)-propansulfonat
Calcium-3-(3-(methyl)butanoylamino)-propansulfonat
Magnesium-3-(3-(methyl)butanoylamino)-propansulfonat
Calcium-3-(2,2-(dimethyl)propanoylamino)-propansulfonat
Magnesium-3-(2,2(dimethyl)propanoylamino)-propansulfonat
Calcium-3-(acetylamino)-2-methylpropansulfonat
Calcium-3-(acetylamino)-3-methylpropansulfonat
Magnesium-3-(acetylamino)-3-methylpropansulfonat
Calcium-3-(acetylamino)-1-methylpropansulfonat
Calcium-3-(acetylamino)-2-phenylpropansulfonat
Calcium-2-(2-acetylaminomethyl)-phenylmethansulfonat
Calcium-N-methyl-3-(acetylamino)-propansulfonat
Calcium-3-(acetylamino)-2,2-dimethylpropansulfonat
Calcium-3-(trifluormethylcarbonyl)-propansulfonat.

3. Verfahren zur Herstellung der Verbindungen mit der Formel I nach Anspruch 1, das darin besteht, eine Verbindung mit der Formel II 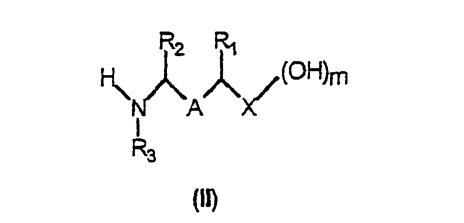 mit einer Verbindung mit der Formel III
**M (OH)**_{**z**} **(III)**
wobei z die Wertigkeit des Metalls M bedeutet, und
anschließend mit einer Verbindung mit der Formel IV 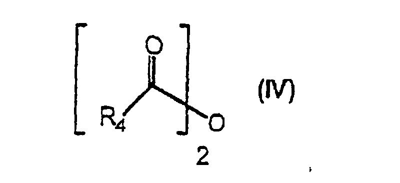 umzusetzen.

4. Pharmazeutische Zusammensetzung, die eine Verbindung nach einem der Ansprüche 1 bis 3 enthält.

## Claims

1. Formula compounds 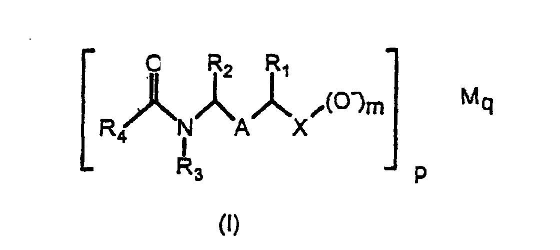 in which:
X represents 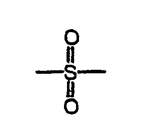
R₁, R₂, R₃ are selected from among hydrogen and a C₁-C₇ alkyl radical.
A represents a formula group 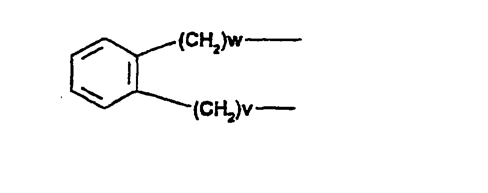 with v and w = 0, 1, 2
or a formula group 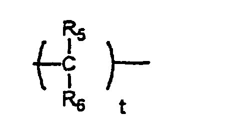
R₅, R₆ being selected independently of each other from among hydrogen, a C₁-C₇ alkyl radical, an aryl radical with 6 to 14 carbon atoms and a hetero-aryl radical selected from among furyl, thienyl and thiazolyl, the aryl and hetero-aryl radicals being capable of bringing in 1 to 3 substitutes selected from among a C₁-C₇ alkyl group, a halogen or a trifluoro methyl group, and t = 1-3.
R4 is selected from among hydrogen, a C₁-C₇ alkyl radical, a CF₃ radical, an aryl radical with 6 to 14 carbon atoms and a hetero-aryl radical selected from among furyl, thienyl and thiazolyl, the aryl and hetero-aryl radicals being capable of bringing in 1 to 3 substitutes selected from among a C₁-C₇ alkyl group, a halogen or a trifluoro methyl group.
M represents a monovalent metal (Na, K, Li) or a divalent metal (Ca, Mg, Sr, Zn), m = 1 or 2
p = 1 - 2 and q = 1 - 2, p and q being such that electrical neutrality of the salt is assured,
R₄ not being a methyl radical when R₁, R₂ and R₃ represent hydrogen.

2. The compounds according to Claim 1, selected from among the following compounds:
3-(2-(methyl) propanoyl amino)-propane sulfonate of calcium
3-(2-(methyl) propanoyl amino)-propane sulfonate of magnesium
3-(butanoyl amino)-propane sulfonate of calcium
3-(butanoyl amino)-propare sulfonate of magnesium
3-(pentanoyl amino)-propane sulfonate of calcium
3-(benzoyl amino)-propane sulfonate of calcium
3-(benzoyl amino)-propane sulfonate of magnesium
3-(2-(methyl) propanoyl amino)-propane sulfonate of zinc
3-(2-(methyl) propanoyl amino)-propane sulfonate of strontium
3-(3-(methyl) butanoyl amino)-propane sulfonate of calcium
3-(3-(methyl) butanoyl amino)-propane sulfonate of magnesium
3-(2-2-(dimethyl) propanoyl amino)-propane sulfonate of calcium
3-(2-2-(dimethyl) propanoyl amino)-propane sulfonate of magnesium
3-(acetyl amino)-2-methyl propane sulfonate of calcium
3-(acetyl amino)-3-methyl propane sulfonate of calcium
3-(acetyl amino)-3-methyl propane sulfonate of magnesium
3-(acetyl amino)-1-methyl propane sulfonate of calcium
3-(acetyl amino)-2-phenyl propane sulfonate of calcium
2-(2-acetyl amino-methyl)-phenyl methane sulfonate of calcium
N-methyl-3-(acetyl amino)-propane sulfonate of calcium
3-(acetyl amino)-2-2-dimethyl propane sulfonate of calcium
3-(trifluoro methyl carbonoyl)-propane sulfonate of calcium

3. Process for preparing the formula I compounds according to Claim 1, which consists in making a formula II compound react: 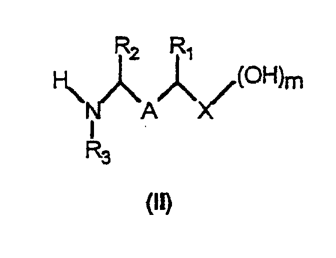 with a formula III compound:
**M(OH)**_{**2**}
z being the valency of the metal M,
and then with a formula IV compound:

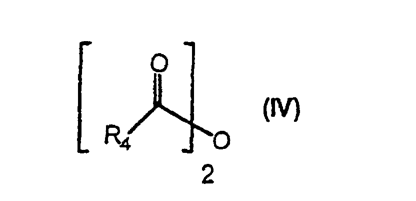

4. Pharmaceutical composition containing a compound according to any one of the Claims 1 to 3.
